# EUROPÄISCHE PATENTSCHRIFT

(11) **EP 2 253 282 B1**
(45) Veröffentlichungstag und Bekanntmachung des Hinweises auf die Patenterteilung: **14.08.2013**
(21) Anmeldenummer: 10005214.1
(22) Anmeldetag: 19.05.2010
(51) Int. Cl.: A61B 17/29, F16C 1/20, A61B 17/295, A61M 1/00, A61B 17/00

(54) **Endoskopischer Manipulator**
Endoscopic manipulator
Manipulateur d'endoscopie

(30) Priorität: 20.05.2009 DE 102009022119
(43) Veröffentlichungstag der Anmeldung: 24.11.2010
(73) Patentinhaber: Karl Storz GmbH & Co. KG, 78532 Tuttlingen (DE)
(72) Erfinder: Hahn, Martin, D-88637 Altheim (DE)
(74) Vertreter: Fugmann, Winfried

(56) Entgegenhaltungen:
- DE-A1- 10 333 342
- DE-A1- 19 520 717
- GB-A- 1 276 384
- US-A- 4 576 167
- US-A- 5 395 364
- US-A- 5 405 348
- US-A1- 2001 021 859
- US-A1- 2005 096 694
- US-A1- 2006 241 630

## Beschreibung

Die vorliegende Erfindung bezieht sich auf einen Manipulator, insbesondere einen endoskopischen Manipulator, für medizinische oder nicht-medizinische technische Zwecke.

Ein Manipulator, insbesondere ein endoskopischer Manipulator für medizinische oder nicht-medizinische technische Anwendungen weist beispielsweise an einem proximalen Ende gegeneinander verschiebbare, verdrehbare oder schwenkbare Griffteile und an einem distalen Ende eine Manipulationseinrichtung auf. Das distale Ende ist mit dem proximalen Ende über einen starren oder flexiblen Schaft verbunden. Wenn die Griffteile am proximalen Ende gegeneinander bewegt werden und/oder eine Kraft oder ein Moment auf die Griffteile wirkt, wird diese Bewegung und/oder diese Kraft bzw. dieses Moment auf die Manipulationseinrichtung übertragen. Dazu ist beispielsweise in dem Schaft des Manipulators ein gegenüber dem Schaft bewegbares Übertragungselement vorgesehen. Wenn der Schaft gekrümmt oder krümmbar ist, muss zumindest in Bereichen, in denen die Krümmung variiert, zumindest entweder der Schaft oder das Übertragungselement flexibel sein, um eine relative Bewegbarkeit zu ermöglichen.

In der EP 1055 397 A1 ist ein medizinisches Instrument zum Abtrennen von Gewebe im menschlichen oder tierischen Körper beschrieben, bei dem eine Spiralfeder einen starren gekrümmten Stab umgibt und gegenüber diesem verschoben wird, um eine Kraft von Griffteilen an einem Ende des Instruments zu einem Werkzeug am anderen Ende des Instruments zu übertragen.

In der EP 1 872 729 A1 ist ein medizinisches Element zum Fassen eines Objekts beschrieben. In einem Schaft zwischen einem Griff und einem distalen Abschnitt ist ein Kraftübertragungselement mit einer helikalen Struktur angeordnet.

Das in der EP 1 872 729 A1 beschriebene helikale Kraftübertragungselement muss biegeelastisch sein und deshalb ein elastisches Material aufweisen, beispielsweise Kunststoff. Damit weist das Kraftübertragungselement jedoch auch in Längsrichtung, in der es eine Kraft übertragen soll, eine hohe Flexibilität bzw. Stauch- und Dehnbarkeit auf. Ferner weist das Kraftübertragungselement eine insgesamt große innen am Schaft anliegende Oberfläche auf. Die resultierende Gleit- oder Haftreibung erschwert ein feinfühliges Arbeiten und erhöht den Verschleiß. Die Schwächung des Querschnitts des Kraftübertragungselements zwischen den Windungen bzw. Gängen seiner helikalen Struktur ist erwünscht, um die Flexibilität zu erhöhen. Gleichzeitig können dort jedoch aufgrund der Kerbwirkung besonders leicht Risse oder andere Schäden im Kraftübertragungselement entstehen, die zu dessen Versagen führen können.

In der US 2001/0021859 A1 ist ein Manipulator mit einer Zange, die mittels eines Antriebsdrahts betätigt wird, beschrieben. Der Antriebsdraht verläuft durch Bohrungen in mehreren Kugeln innerhalb eines Hohlraums eines elastischen Bauglieds. Die zweiteilige

Form der Ansprüche 1 und 8 ist auf diesem Dokument basiert.

In der DE 103 33 342 A1 ist ein chirurgisches Rohrschaftinstrument beschrieben. In einem hohlen Schaft ist ein Kraftübertragungselement zum Übertragen einer Kraft von einer Grifibranche zu einem Arbeitselement angeordnet. Das Kraftübertragungselement umfasst eine Reihe von Einzelabschnitten, die über Kugelgelenke miteinander verbunden sind, und ein flexibles Zugglied innerhalb der Einzelabschnitte.

In der US 5,395,364 A ist ein endoskopisches Instrument mit einer Elastomer-Fluiddichtung beschrieben. Innerhalb eines Rohrs ist eine Schubstange zum Übertragen einer Kraft von einer Betätigungseinrichtung zu einer Wirkeinrichtung angeordnet. Um zu verhindern, dass innerhalb des Rohres ein Fluid fließen kann, ist eine Elastomer-Dichtung zwischen der Innenwand des Rohrs und der Schubstange angeordnet.

In der US 4,576,167 A ist eine chirurgische Klammereinrichtung mit einem gebogenen Schaft beschrieben. Zur Übertragung einer Kraft von einer Betätigungsanordnung zu einer Klammeranordnung ist innerhalb des gebogenen Schafts ein flexibles Band angeordnet. Zur Führung des flexiblen Bands ist eine Abstandseinrichtung ähnlich einem Linear-Kugellager vorgesehen.

In der US 2005/0096694 A1 ist ein chirurgisches Instrument mit einem krümmbaren Abschnitt beschrieben. In dem krümmbaren Abschnitt weist das chirurgische Instrument alternierend Rippen bzw. Lamellen und Aussparungen bzw. Einschnitte auf. In den Lamellen bzw. Rippen sind eine Schubstange zur Übertragung einer Kraft zu einem Werkzeug und Steuerkabel zum Steuern einer Krümmung geführt.

In der GB 1 276 384 ist eine Fernsteuerungsanordnung zur Bewegungsübertragung beschrieben. In einem äußeren Rohr ist ein Innenelement längsverschiebbar durch von einem Käfig gehaltene Kugeln geführt.

In der US 2006/0241630 A1 ist ein chirurgisches Schneidinstrument beschrieben. Das Schneidinstrument umfasst ein gekrümmtes Außenrohr, in dem eine Drahtanordnung zum Übertragen einer Rotationsbewegung angeordnet und durch eine Lagerhülse geführt ist.

In der US 5,405,384 A ist ein chirurgisches Schneidinstrument beschrieben. Das chirurgische Schneidinstrument umfasst ein Rohr, in dem ein Schaft eines Schneidewerkzeugs durch punktförmige oder linienförmige Anlage an Lagerelementen oder in einem zylindrischen Lager geführt ist.

In der DE 195 20 717 A1 ist ein chirurgisches Rohrschaftinstrument beschrieben. Im Inneren eines Schafts ist zur Übertragung einer Kraft ein Stab aus einem biegeelastischen Material mit Umfangsnuten zwischen kurzen Abschnitten mit nicht reduziertem Durchmesser vorgesehen.

Eine Aufgabe der vorliegenden Erfindung besteht darin, einen verbesserten Manipulator zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Idee, im Schaft eines Manipulators einen Führungseinsatz vorzusehen, der eine Zug- oder Druckstange als Übertragungselement führt. Da die Zug- oder Druckstange durch den Führungseinsatz geführt wird, kann sie einen geringen und in Längsrichtung zumindest abschnittsweise konstanten bzw. ortsunabhängigen Querschnitt aufweisen. Aufgrund des geringen Querschnitts kann die Zug- oder Druckstange Stahl oder ein anderes Material mit einer geringen Elastizität aufweisen. Die Zug- oder Druckstange ist damit ein Übertragungselement mit einer deutlich geringeren Stauch- oder Dehnbarkeit als beispielsweise das in der EP 1 872 729 A1 beschriebene helikale Kraftübertragungselement.

Durch den möglichen geringen Querschnitt der Zug- oder Druckstange bzw. des Übertragungselements wird die Reibung, insbesondere die Haftreibung zwischen dem Übertragungselement und dem Schaft bzw. dem Führungseinsatz im Schaft verringert. Dadurch wird ein feinfühligeres Arbeiten mit dem Manipulator ermöglicht. Dies ist besonders für medizinische aber auch für andere nicht-medizinische technische Anwendungen vorteilhaft. Durch die verringerte Reibung wird auch der Verschleiß verringert.

Der mögliche konstante Querschnitt des Übertragungselements mindert ferner das Risiko einer Schädigung des Übertragungselements durch Risse oder auf andere Weise, da mangels einer Kerbwirkung keine lokalen Spannungsmaxima auftreten.

Ein Manipulator umfasst ein proximales Ende, ein distales Ende mit einer Manipulationseinrichtung, einen Schaft zwischen dem proximalen Ende und dem distalen Ende und ein Übertragungselement zur Übertragung zumindest entweder einer Kraft oder einer Bewegung zwischen dem proximalen Ende und dem distalen Ende des Schafts. In dem Schaft ist ein Führungseinsatz zum Führen des Übertragungselements vorgesehen, wobei das Übertragungselement gegenüber dem Führungseinsatz in Längsrichtung des Schafts verschiebbar ist, und wobei der Führungseinsatz einstückig ausgebildet ist.

Das Übertragungselement ist insbesondere eine Zug- oder Druckstange. Der Querschnitt des Übertragungselements kann zwischen dem proximalen Ende und dem distalen Ende konstant bzw. im Wesentlichen konstant oder abschnittsweise im Wesentlichen konstant sein. Der Führungseinsatz weist insbesondere einen zentrischen oder exzentrischen Kanal auf, in dem das Übertragungselement verschiebbar angeordnet ist.

Zur Erhöhung der Flexibilität des Führungseinsatzes weist dieser in Längsrichtung alternierend Stützabschnitte und flexible Abschnitte auf. Die Stützabschnitte sind ausgebildet, um an der Innenwand des Schafts anzuliegen. Die flexiblen Abschnitte können insbesondere einen gegenüber den Stützabschnitten deutlich reduzierten Querschnitt aufweisen. Alternativ oder zusätzlich können die flexiblen Abschnitte ein Material aufweisen, das elastischer ist als ein Material der Stützabschnitte. Die Stützabschnitte können Spüldurchlässe aufweisen, durch die ein Spülfluid in Längsrichtung des Schafts fließen kann. Alternativ zur alternierenden Anordnung von Stützabschnitten und flexiblen Abschnitten kann der Führungseinsatz eine helikale Struktur aufweisen. Ferner weist der Führungseinsätz einen Kanal für das Übertragungselement auf, der über die gesamte Länge des Führungseinsatzes oder auch nur abschnittsweise vollständig innerhalb des Querschnitts des Führungseinsatzes angeordnet sein kann.

Das Übertragungselement und der Führungseinsatz können zwei verschiedene Materialien aufweisen. Beispielsweise kann das Übertragungselement Edelstahl oder ein anderes Material mit einer geringen Elastizität aufweisen, um eine geringe Stauch- bzw. Dehnbarkeit des Übertragungselements zu gewährleisten. Der Führungseinsatz kann beispielsweise Polyetheretherketon (PEEK; ein aromatischer, teilkristalliner Thermoplast aus der Gruppe der Polyaryletherketone) oder ein anderes Polyaryletherketon (PAEK) oder ein anderer Thermoplast oder PTFE (auch unter dem Markennamen Teflon bekannt) aufweisen.

Der Schaft des Manipulators kann gekrümmt oder aufgrund eigener Elastizität oder aufgrund von Gelenken krümmbar sein. Die Manipulationseinrichtung kann zumindest entweder eine Zange oder einen Greifer oder ein Schneidewerkzeug oder ein Stanzwerkzeug umfassen. Der Manipulator ist insbesondere ein endoskopischer Manipulator für medizinische oder nicht-medizinische technische Anwendungen.

Ein Führungseinsatz für einen Manipulator mit einem Schaft zwischen einem proximalen Ende und einem distalen Ende umfasst zumindest einen Abschnitt mit einem an einen Hohlraum des Schafts angepassten Querschnitt und einen Kanal in dem ein Übertragungselement zur Übertragung zumindest entweder einer Kraft oder einer Bewegung zwischen dem proximalen und dem distalen Ende des Schafts verschiebbar angeordnet werden kann, wobei der Führungseinsatz einstückig ausgebildet ist und in seiner Längsrichtung altemierend Stützabschnitte und flexible Abschnitte aufweist. Der Führungseinsatz kann die oben bereits beschriebenen Merkmale aufweisen.

Die Erfindung ist beispielsweise für einen Uterusmanipulator anwendbar.

### Kurzbeschreibung der Figuren

Nachfolgend werden Ausführungsformen anhand der beigefügten Figuren näher erläutert. Es zeigen:
- Figur 1: eine schematische Darstellung eines endoskopische Manipulators;
- Figur 2: eine schematische Darstellung eines teilweise aufgeschnittenen Schafts eines Manipulators;
- Figur 3: eine schematische Darstellung eines Führungseinsatzes;
- Figur 4: eine schematische Darstellung eines Querschnitts eines Führungseinsatzes; und
- Figur 5: eine schematische Darstellung eines Querschnitts eines weiteren Führungseinsatzes.

### Beschreibung der Ausführungsformen

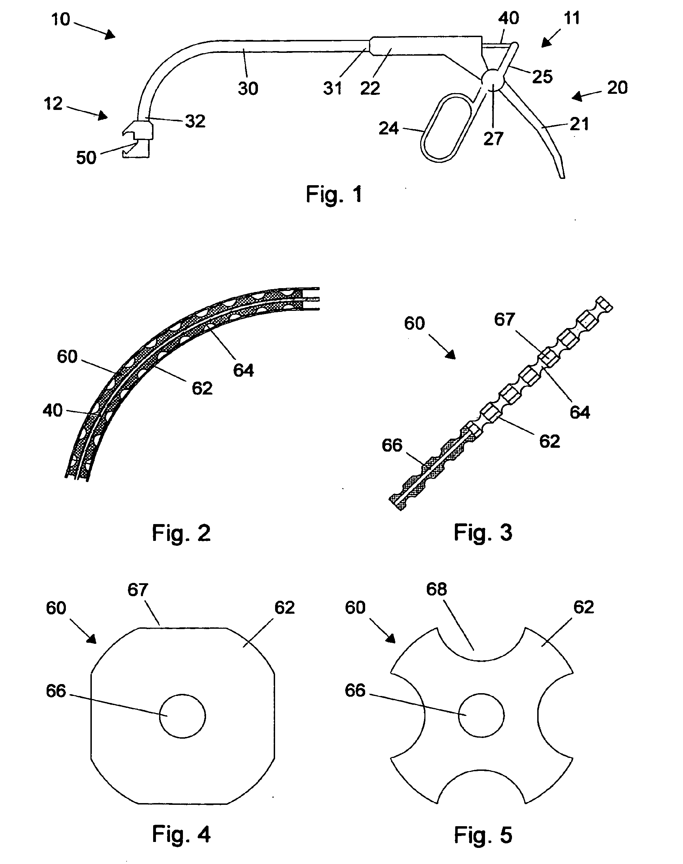

Figur 1 zeigt eine schematische Darstellung eines Manipulators 10, insbesondere eines endoskopischen Manipulators für medizinische oder nicht-medizinische technische Anwendungen. Der Manipulator 10 umfasst an einem proximalen Ende 11 eine Handhabungseinrichtung 20. Die Handhabungseinrichtung 20 umfasst ein feststehendes Griffteil 21, ein Verbindungselement 22, ein bewegbares Griffteil 24, einen Hebel 25 und ein Gelenk 27. Das feststehende Griffteil 21 ist starr mit dem Verbindungselement 22 verbunden. Der Hebel 25 ist starr mit dem bewegbaren Griffteil 24 verbunden. Das Gelenk 27 verbindet das feststehende Griffteil 21 und das Verbindungselement 22 einerseits sowie das bewegbare Griffteil 24 und den Hebel 25 andererseits so, dass das bewegbare Griffteil 24 und der Hebel 25 gegenüber dem feststehenden Griffteil 21 und dem Verbindungselement 22 um eine Achse rotierbar bzw. schwenkbar sind, die senkrecht oder im Wesentlichen senkrecht zur Zeichenebene von Figur 1 steht. Das feststehende Griffteil 21 und das bewegbare Griffteil 24 sind so ausgebildet, dass eine Person mit einer Hand das feststehende Griffteil 21 und das bewegbare Griffteil 24 umgreifen, damit die Handhabungseinrichtung 20 halten, den Manipulator 10 führen und gleichzeitig das feststehende Griffteil 21 und das bewegbare Griffteil 24 gegeneinander bewegen kann.

Alternativ kann das bewegliche Griffteil auch gegenüber dem feststehenden Griffteil in diesem oder um dieses verdreht werden oder gegenüber dem feststehenden Griffteil translatorisch, ggf. über gekrümmt verlaufende oder allgemein nicht gerade Strecken, verschoben werden.

Der Manipulator 10 umfasst ferner einen Schaft 30 mit einem proximalen Ende 31 und einem distalen Ende 32. Das proximale Ende 31 des Schafts 30 ist mit dem Verbindungselement 22 der Handhabungseinrichtung 20 verbunden, insbesondere starr verbunden. Der Schaft 30 wird insbesondere durch ein Rohr gebildet, das starr oder zumindest abschnittsweise flexibel sein kann.

Der Manipulator 10 umfasst ferner ein Übertragungselement 40, insbesondere in Form eines dünnen und zumindest abschnittsweise biegbaren Stabs, der in dem Schaft 30 angeordnet ist. Das Übertragungselement 40 ragt am proximalen Ende 11 des Manipulators aus dem Verbindungselement 22 heraus. Das Ende des Übertragungselements 40 ist dort gelenkig mit dem Hebel 25 der Handhabungseinrichtung 20 gekoppelt.

Der Manipulator 10 umfasst ferner an seinem distalen Ende 12 eine Manipulationseinrichtung 50, die mit dem distalen Ende 32 des Schafts 30 verbunden ist. Die Manipulationseinrichtung 50 ist oder umfasst beispielsweise eine Zange oder einen Greifer oder ein Schneidewerkzeug oder ein Stanzwerkzeug oder einen Spreizer oder Dilatator. Die Manipulationseinrichtung 50 ist in Figur 1 nur schematisch dargestellt.

Das Übertragungselement 40 ist im Schaft 30 längs verschiebbar, am proximalen Ende 11 des Manipulators 10 mit dem Hebel 25 und am distalen Ende 12 des Manipulators 10 mit der Manipulationseinrichtung 50 verbunden oder gekoppelt. Eine relative Bewegung des bewegbaren Griffteils 24 gegenüber dem feststehenden Griffteil 21 und wird über eine entsprechende relative Bewegung des Übertragungselements 40 gegenüber dem Schaft 30 zur Manipulationseinrichtung 50 übertragen. Entsprechend wird eine Kraft auf das feststehende Griffteil 21 und das bewegbare Griffteil 24 bzw. eine gegenseitige Verspannung des feststehenden Griffteils 21 und des bewegbaren Griffteils 24 durch den Schaft 30 und das Übertragungselement 40 auf die Manipulationseinrichtung 50 übertragen.

Figur 2 zeigt eine schematische Darstellung eines Längsschnitts des Schafts 30 in einem gekrümmten Bereich. Die Schnittebene liegt parallel zur Zeichenebene der Figur 1.

Im rohrförmigen Schaft 30 ist ein Führungseinsatz 60 angeordnet, der unten mit Bezug auf Figur 3 näher beschrieben wird. Im Führungseinsatz 60 ist das Übertragungselement 40 angeordnet. Der Führungseinsatz 60 weist Stützabschnitte 62 und flexible Abschnitte 64 auf. Die Stützabschnitte 62 liegen an der Innenwand des rohrförmigen Schafts 30 an. Die flexiblen Abschnitte 64 weisen einen verringerten Querschnitt auf und erlauben eine Krümmung des Führungseinsatzes 60 entsprechend der Krümmung des Schafts 30. Aufgrund des geringeren Querschnitts sind die flexiblen Abschnitte 64 leichter elastisch oder auch plastisch verformbar als die Stützabschnitte 62. Dennoch können auch die Stützabschnitte 62 zur Biegeelastizität des Führungseinsatzes 60 beitragen.

Das Übertragungselement 40 ist in einem Kanal 66 angeordnet. Der Kanal 66 ist insbesondere zentrisch bzw. in der Mitte des Querschnitts des Führungseinsatzes 60 angeordnet. Der Querschnitt des Kanals 66 ist so an den Querschnitt des Übertragungselements 40 angepasst, dass das Übertragungselement 40 in dem Kanal 66 einerseits in Längsrichtung reibungsarm bewegbar und andererseits in transversaler Richtung spielarm geführt ist.

Figur 3 zeigt eine schematische Darstellung des bereits oben anhand der Figur 2 dargestellten Führungseinsatzes 60. Dabei sind in dem in Figur 3 rechts oben dargestellten Bereich eine Draufsicht auf den Führungseinsatz 60 und in dem in Figur 3 links unten dargestellten Bereich ein Längsschnitt des Führungseinsatzes 60 gezeigt. Die Schnittebene des links unten gezeigten Schnitts entspricht der Schnittebene der Figur 2.

Der Führungseinsatz 60 ist in Figur 3 in einer ungekrümmten bzw. geraden Form dargestellt, in der er beispielsweise hergesellt wird. Der Führungseinsatz 60 weist beispielsweise Polyetheretherketon (PEEK; ein aromatischer, teilkristalliner Thermoplast aus der Gruppe der Polyaryletherketone) oder ein anderes Polyaryletherketon (PAEK) oder ein anderer Thermoplast oder PTFE (auch unter dem Markennamen Teflon bekannt) oder ein anderes elastisches Material auf.

Der Führungseinsatz 60 weist in Längsrichtung alternierend die bereits oben anhand der Figur 2 dargestellten Stützabschnitte 62 und flexiblen Abschnitte 64 auf. Die Stützabschnitte 62 weisen einen Querschnitt auf, der an den Querschnitt eines Hohlraums im Schaft 30 angepasst ist, so dass die Stützabschnitte 62 transversal spielarm oder spielfrei im Schaft 30 geführt oder dort sogar elastisch geklemmt sind. Die flexiblen Abschnitte 64 weisen einen gegenüber den Querschnitten der Stützabschnitte 62 und dem Querschnitt des Hohlraums im Schaft 30 geringeren oder wesentlich geringeren Querschnitt auf. Die Stützabschnitte 62 weisen ferner Abflachungen 67 auf, die unten mit Bezug auf Figur 4 näher beschrieben werden. Der Kanal 66 liegt über die gesamte Länge des Führungseinsatzes 60 vollständig innerhalb des Querschnitts der Stützabschnitte 62 des Führungseinsatzes 60.

Die Figuren 4 und 5 zeigen schematische Darstellungen von Querschnitten von Stützabschnitten 62 von Führungseinsätzen 60 ähnlich dem oben anhand der Figuren 2 und 3 beschriebenen. Die Schnittebenen liegen dabei senkrecht zur Längsrichtung des oben anhand der Figuren 1 und 2 beschriebenen Schafts 30 und des oben anhand der Figuren 1 und 2 dargestellten Übertragungselements 40 und senkrecht zu den Zeichenebenen der Figuren 1 bis 3.

Figur 4 zeigt einen Querschnitt eines Stützabschnitts 62 eines Führungseinsatzes 60, der von einer reinen Kreisform durch Abflachungen 67 abweicht. Figur 5 zeigt einen Querschnitt eines Stützabschnitts 62 eines Führungseinsatzes 60, der von einer reinen Kreisform durch Nuten 68 abweicht. Die Abflachungen 67 bzw. Nuten 68 erlauben ein Fließen bzw. Strömen eines Spülfluids, insbesondere einer Spülflüssigkeit, in Längsrichtung des Schafts zwischen einer Innenwand des Schafts und dem Führungseinsatz 60.

In den Figuren 4 und 5 ist jeweils eine regelmäßige Anordnung von vier Abflachungen 67 bzw. vier Nuten 68 dargestellt. Abweichend davon sind auch jede andere Anzahl von Abflachungen 67 bzw. Nuten 68 und eine unregelmäßige Anordnung der Abflachungen 67 bzw. Nuten 68 möglich. Auch der Querschnitt der Nut 68 kann von der Darstellung in Figur 5 abweichen. Insbesondere kann der Querschnitt der Nut 68 die Form eines Kreissegments, eines Trapezes, eines Rechtecks oder eine beliebige andere Form aufweisen.

Der Kanal 66 ist in den Figuren 2 bis 5 jeweils zentrisch bzw. koaxial zum äußeren Umfang des Führungseinsatzes 60 und zum rohrförmigen Schaft 30 dargestellt. Stattdessen ist auch eine exzentrische Anordnung des Kanals 66 möglich. Abweichend von den Darstellungen anhand der Figuren sind ferner nicht-kreisförmige Querschnitte des Schafts 30 und des Übertragungselements 40 möglich. Anstelle der oben anhand der Figuren dargestellten alternierenden Anordnung von Stützabschnitten 62 und flexiblen Abschnitten 64 ist ferner beispielsweise eine helikale Struktur des Führungseinsatzes möglich. Bei einer helikalen Struktur des Führungseinsatzes 60 verbleibt zwischen einem oder mehreren schraubenförmig verlaufenden Stegen des Führungseinsatzes 60, die an der Innenwand des Hohlraums im Schaft 30 anliegen, eine entsprechende Anzahl von schraubenförmigen Kanälen, die ein Strömen bzw. Fließen eines Spülfluids in Längsrichtung des Schafts 30 ermöglichen.

Ein Führungseinsatz 60, wie er oben anhand der Figuren 2 bis 5 beschrieben wurde, kann lediglich wie in Figur 2 angedeutet, in einem gekrümmten Bereich des Schafts 30 angeordnet sein. Während das Übertragungselement 40 in jedem Fall innerhalb des Führungseinsatzes 60 vorteilhaft einen konstanten Querschnitt aufweist bzw. zylindersymmetrisch (translationssymmetrisch zur Längsrichtung) ist, kann in diesem Fall das Übertragungselement 40 außerhalb des Führungseinsatzes 60 einen anderen Querschnitt aufweisen. Alternativ kann der Führungseinsatz 60 eine größere Länge aufweisen und auch in geraden Abschnitten des Schafts 30 angeordnet sein oder sich über die gesamte Länge des Schafts 30 erstrecken. Anders als oben anhand der Figuren 2 bis 5 dargestellt, kann der Kanal 66 ferner alternativ nur abschnittsweise innerhalb oder vollständig innerhalb des Querschnitts des Führungseinsatzes 60 angeordnet sein. Beispielsweise kann das Übertragungselement 40 nur in den Bereichen der Stützabschnitte 62 innerhalb der Stützabschnitte 62 des Führungseinsatzes 60 und bei den flexiblen Abschnitten 64 außerhalb des Führungseinsatzes 60 verlaufen.

Bezugszeichen
- 10: Manipulator
- 11: proximales Ende des Manipulators 10
- 12: distales Ende des Manipulators 10
- 20: Handhabungseinrichtung des Manipulators 10
- 21: feststehendes Griffteil
- 22: Verbindungselement
- 24: bewegbares Griffteil
- 25: Hebel
- 27: Gelenk
- 30: Schaft
- 31: proximales Ende des Schafts 30
- 32: distales Ende des Schafts 30
- 40: Übertragungselement
- 41: proximales Ende des Übertragungselements 40
- 42: distales Ende des Übertragungselements 40
- 50: Manipulationseinrichtung
- 60: Führungseinsatz
- 62: Stützabschnitt des Führungseinsatzes 60
- 64: flexibler Abschnitt des Führungseinsatzes 60
- 66: Kanal des Führungseinsatzes 60
- 67: Abflachung an einem Stützabschnitt 66
- 68: Nut an einem Stützabschnitt 66

## Patentansprüche

1. **Manipulator** (10) mit:
einem **proximalen Ende** (11);
einem **distalen Ende** (12) mit einer Manipulationseinrichtung (50);
einem **Schaft** (30) zwischen dem proximalen Ende (11) und dem distalen Ende (12);
einem **Übertragungselement** (40) zur Übertragung zumindest entweder einer Kraft oder einer Bewegung zwischen dem proximalen Ende (11) und dem distalen Ende (12);
einem **Führungseinsatz** (60) in dem Schaft (30), zum Führen des Übertragungselements (40), wobei der Führungseinsatz (60) einen **Kanal** (66) aufweist. in dem das Übertragungselement (40) verschiebbar angeordnet ist, dardurch gekennzeichnet dass der Führungseinsatz (60) einstückig ausgebildet ist und in seiner Längsrichtung alternierend **Stützabschnitte** (62) und **flexible Abschnitte** (64) aufweist.

2. Manipulator (10) nach dem vorangehenden Anspruch, wobei die Stützabschnitte (62) an einer Innenwand des Schafts (30) anliegen, und wobei die flexiblen Abschnitte (64) **Querschnitte** aufweisen, die geringer oder wesentlich geringer sind als ein Innenquerschnitt des Schafts (30).

3. Manipulator (10) nach dem vorangehenden Anspruch, wobei die Stützabschnitte (62) **Spüldurchlässe** (67; 68) aufweisen, durch die ein Spülfluid in Längsrichtung des Schafts (30) fließen kann.

4. Manipulator (10) nach einem der vorangehenden Ansprüche, wobei das Übertragungselement (40) und der Führungseinsatz (60) **verschiedene Materialien** aufweisen.

5. Manipulator (10) nach einem der vorangehenden Ansprüche, wobei der Querschnitt des Übertragungselements (40) zwischen dem proximalen Ende (11) und dem distalen Ende (12) konstant ist.

6. Manipulator (10) nach einem der vorangehenden Ansprüche, wobei die Manipulationseinrichtung (50) zumindest entweder eine **Zange** oder einen **Greifer** oder ein **Schneidewerkzeug** oder ein **Stanzwerkzeug** umfasst.

7. Manipulator (10) nach einem der vorangehenden Ansprüche, wobei der Manipulator (10) ein **endoskopischer** Manipulator ist.

8. **Führungseinsatz** (60) für einen Manipulator (10) mit einem Schaft (30) zwischen einem proximalen Ende (11) und einem distalen Ende (12), mit
zumindest einem Abschnitt (62) mit einem an einen Hohlraum des Schafts (30) angepassten Querschnitt;
einem **Kanal** (66), in dem ein Übertragungselement (40) zur Übertragung zumindest entweder einer Kraft oder einer Bewegung zwischen dem proximalen Ende (11) und dem distalen Ende (12) verschiebbar angeordnet werden kann,
**dadurch gekennzeichnet dass** der Führungseinsatz (60) einstückig ausgebildet ist und in seiner Längsrichtung alternierend **Stützabschnitte** (62) und **flexible Abschnitte** (64) aufweist.

9. Führungseinsatz (60) nach Anspruch 8, wobei die Stützabschnitte (62) ausgebildet sind, um an einer Innenwand des Schafts (30) anzuliegen, und wobei die flexiblen Abschnitte (64) Querschnitte aufweisen, die wesentlich geringer sind als ein Innenquerschnitt des Schafts (30).

10. Führungseinsatz (60) nach dem vorangehenden Anspruch, wobei die Stützabschnitte (62) **Spüldurchlässe** (67; 68) aufweisen, durch die ein Spülfluid in Längsrichtung des Schafts (30) fließen kann.

## Claims

1. Manipulator (10) with:
a proximal end (11);
a distal end (12) with a manipulating device (50);
a shank (30) between the proximal end (11) and the distal end (12);
a transmission element (40) for transmitting at least either a force or a movement between the proximal end (11) and the distal end (12);
a guide insert (60) arranged in the shank (30) for the purpose of guiding the transmission element (40), wherein the guide insert (60) has a channel (66) in which the transmission element (40) is arranged movably, **characterized in that** the guide insert (60) is formed in one piece and, in its longitudinal direction, has alternating support portions (62) and flexible portions (64).

2. Manipulator (10) according to the preceding claim, wherein the support portions (62) bear on an inner wall of the shank (30), and wherein the flexible portions (64) have cross sections that are smaller or substantially smaller than an internal cross section of the shank (30).

3. Manipulator (10) according to the preceding claim, wherein the support portions (62) have irrigation openings (67; 68) through which an irrigation fluid can flow in the longitudinal direction of the shank (30).

4. Manipulator (10) according to one of the preceding claims, wherein the transmission element (40) and the guide insert (60) are made of different materials.

5. Manipulator (10) according to one of the preceding claims, wherein the cross section of the transmission element (40) is constant between the proximal end (11) and the distal end (12).

6. Manipulator (10) according to one of the preceding claims, wherein the manipulating device (50) comprises at least either forceps or a gripper or a cutting tool or a punching tool.

7. Manipulator (10) according to one of the preceding claims, wherein the manipulator (10) is an endoscopic manipulator.

8. Guide insert (60) for a manipulator (10) with a shank (30) between a proximal end (11) and a distal end (12), with
at least one portion (62) with a cross section adapted to a cavity of the shank (30);
a channel (66) in which a transmission element (40) for transmitting at least either a force or a movement between the proximal end (11) and the distal end (12) can be arranged movably, **characterized in that** the guide insert (60) is formed in one piece and, in its longitudinal direction, has alternating support portions (62) and flexible portions (64).

9. Guide insert (60) according to Claim 8, wherein the support portions (62) are designed to bear on an inner wall of the shank (30), and wherein the flexible portions (64) have cross sections that are substantially smaller than an internal cross section of the shank (30).

10. Guide insert (60) according to the preceding claim, wherein the support portions (62) have irrigation openings (67; 68) through which an irrigation fluid can flow in the longitudinal direction of the shank (30).

## Revendications

1. Manipulateur (10) comprenant :
une extrémité proximale (11) ;
une extrémité distale (12) avec un dispositif de manipulation (50) ;
une tige (30) entre l'extrémité proximale (11) et l'extrémité distale (12) ;
un élément de transfert (40) pour transférer au moins soit une force soit un mouvement entre l'extrémité proximale (11) et l'extrémité distale (12) ;
un insert de guidage (60) dans la tige (30), pour guider l'élément de transfert (40), l'insert de guidage (60) présentant un canal (66), dans lequel est disposé de manière déplaçable l'élément de transfert (40), **caractérisé en ce que** l'insert de guidage (60) est réalisé d'une seule pièce et présente des portions de support (62) et des portions flexibles (64) alternant dans sa direction longitudinale.

2. Manipulateur (10) selon la revendication précédente, dans lequel les portions de support (62) s'appliquent contre une paroi interne de la tige (30), et dans lequel les portions flexibles (64) présentent des sections transversales qui sont inférieures ou sensiblement inférieures à une section transversale interne de la tige (30).

3. Manipulateur (10) selon la revendication précédente, dans lequel les portions de support (62) présentent des passages de rinçage (67 ; 68), à travers lesquels peut s'écouler un fluide de rinçage dans la direction longitudinale de la tige (30).

4. Manipulateur (10) selon l'une quelconque des revendications précédentes, dans lequel l'élément de transfert (40) et l'insert de guidage (60) présentent des matériaux différents.

5. Manipulateur (10) selon l'une quelconque des revendications précédentes, dans lequel la section transversale de l'élément de transfert (40) est constante entre l'extrémité proximale (11) et l'extrémité distale (12).

6. Manipulateur (10) selon l'une quelconque des revendications précédentes, dans lequel le dispositif de manipulation (50) comprend au moins soit une pince soit un élément de préhension soit un outil de coupe soit un outil d'estampage.

7. Manipulateur (10) selon l'une quelconque des revendications précédentes, dans lequel le manipulateur (10) est un manipulateur endoscopique.

8. Insert de guidage (60) pour un manipulateur (10) comprenant une tige (30) entre une extrémité proximale (11) et une extrémité distale (12), avec au moins une portion (62) ayant une section transversale adaptée à une cavité de la tige (30) ;
un canal (66), dans lequel un élément de transfert (40) peut être disposé de manière déplaçable pour le transfert d'au moins soit une force soit un mouvement entre l'extrémité proximale (11) et l'extrémité distale (12),
**caractérisé en ce que**
l'insert de guidage (60) est réalisé d'une seule pièce et présente des portions de support (62) et des portions flexibles (64) alternant dans sa direction longitudinale.

9. Insert de guidage (60) selon la revendication 8, dans lequel les portions de support (62) sont réalisées de manière à s'appliquer contre une paroi intérieure de la tige (30), et dans lequel les portions flexibles (64) présentent des sections transversales qui sont sensiblement inférieures à une section transversale interne de la tige (30).

10. Insert de guidage (60) selon la revendication précédente, dans lequel les portions de support (62) présentent des passages de rinçage (67 ; 68), à travers lesquels un fluide de rinçage peut s'écouler dans la direction longitudinale de la tige (30).
